# EUROPEAN PATENT SPECIFICATION

(11) **EP 4 205 584 B1**
(45) Date of publication and mention of the grant of the patent: **08.10.2025**
(21) Application number: 20950911.6
(22) Date of filing: 31.08.2020
(51) Int. Cl.: A24F 40/40, A61M 11/04, A61M 15/06, A24F 40/10, A24F 40/48

(54) **ELECTRONIC ATOMIZING ASSEMBLY AND DEVICE THEREOF**
ELEKTRONISCHE ZERSTÄUBUNGSANORDNUNG UND VORRICHTUNG DAFÜR
ENSEMBLE D'ATOMISATION ÉLECTRONIQUE ET DISPOSITIF ASSOCIÉ

(43) Date of publication of application: 05.07.2023
(73) Proprietor: Shenzhen Smoore Technology Limited, Shenzhen, Guangdong 518102 (CN)
(72) Inventor: LEI, Guilin, Shenzhen, Guangdong 518102 (CN); JIANG, Ru, Shenzhen, Guangdong 518102 (CN); CAO, Rui, Shenzhen, Guangdong 518102 (CN)
(74) Representative: Herrero & Asociados, S.L.
(86) International application number: PCT/CN2020/112674
(87) International publication number: WO 2022/041242

(56) References cited:
- CN-A- 105 105 339
- CN-A- 105 105 339
- CN-A- 110 403 246
- CN-A- 110 623 308
- CN-A- 110 893 016
- CN-A- 111 109 664
- CN-U- 210 471 007
- KR-A- 20200 070 268
- US-A1- 2019 231 991

## Description

### TECHNICAL FIELD

The present disclosure relates to the technical field of atomizers, and in particular the present invention relates to an electronic atomizing assembly and an electronic atomizing device.

### BACKGROUND

An electronic atomizing device includes an electronic atomizing assembly and a power supply component, where the electronic atomizing assembly is configured to atomize e-liquid, and the power supply component is configured to supply power to the electronic atomizing assembly. In the electronic atomizing assembly, when an amount of e-liquid flowing into an atomizing core from a liquid storage tank through a liquid flowing channel is excessively large, a liquid leakage problem may be caused; when the amount of the e-liquid is excessive small, problems such as e-liquid explosion and a burnt flavor may be caused. That is, a liquid-supplying amount has a significant impact on the performance of the electronic atomizing device.

CN105105339A relates to an electronic cigarette atomizer and an electronic cigarette. The electronic cigarette atomizer includes a smoking oil cavity shell, a smoking oil cavity base connected with the smoking oil cavity shell, an atomization sucker and an atomization core, wherein the atomization sucker and the atomizing core are located in the smoking oil cavity shell. An atomized gas outlet hole is formed in the smoking oil cavity shell. The atomization core includes an atomization cavity shell, an atomization core base, a hollow cylindrical oil sucking body and a heating wire. The atomization cavity shell is connected with the atomization core base. The two ends of the atomization sucker are connected with the smoking oil cavity shell and the atomization cavity shell respectively. The heating wire is a double-spiral-ring heating wire with two free ends. The double-spiral ring is of the structure that inner ring bodies and outer ring bodies which are coaxial are arranged in a sleeved mode. The inner ring bodies and the outer ring bodies of the double-spiral ring of the heating wire are alternately embedded into the oil sucking body in the axial direction. Micro holes for smoking oil to permeate are formed in the atomization cavity shell. First air inlets are formed in the smoking oil cavity base. A through hole is formed in the atomization core base. Air flow circulation is formed among the first air inlets, the through hole, the atomization sucker and the atomized gas outlet hole.

### SUMMARY OF THE INVENTION

The present invention provides an electronic atomizing assembly as set forth in claim 1 and an electronic atomizing device as set forth in claim 12, to resolve a problem of controlling the liquid-supplying amount in the related art.

To resolve the foregoing technical problem, a first technical solution provided in the present invention is to provide an electronic atomizing assembly. The electronic atomizing assembly includes an atomizing core, an atomizing base, and a liquid storage tank. The atomizing core is configured to atomize liquid and includes a heating body and an atomizing core body, and the atomizing core body has a porous structure. The atomizing base includes an atomizing top base, an atomizing bottom base, and a microgroove structure. The atomizing core is fixed between the atomizing top base and the atomizing bottom base. The atomizing top base is cooperated with the atomizing bottom base to form an atomizing cavity. The microgroove structure includes a plurality of microgrooves. The surface of at least one of the atomizing top base and the atomizing bottom base which is configured to be contacted with the atomizing core body is provided with the plurality of microgrooves, and the width of each of the plurality of microgrooves is less than 1 mm. The liquid storage tank is sleeved on the atomizing base and configured to store the liquid.

To resolve the foregoing technical problem, a second technical solution provided in the present invention is to provide an electronic atomizing device. The electronic atomizing device includes an electronic atomizing assembly described in any of the above embodiments and a power supply component.

Beneficial effects of the present disclosure are as follows: Different from the related art, in the present disclosure, since a microgroove structure is provided on the surface of an atomizing base which is configured to be contacted with an atomizing core body, the liquid-supplying amount of the e-liquid entering an atomizing core may be adjusted finely. In this way, a precise control for liquid-supplying amounts of different products may be achieved, and the liquid leakage problem caused by the liquid-supplying amount being excessively large and the problems such as the e-liquid explosion and the burnt flavor caused by the liquid-supplying amount being excessively small may be avoided. Therefore, the performance of the electronic atomizing device may be improved.

### BRIEF DESCRIPTION OF THE DRAWINGS

To describe the technical solutions in the embodiments of the present disclosure more clearly, the accompanying drawings required for describing the embodiments will be introduced briefly in the following. Apparently, the accompanying drawings described in the following only show some embodiments of the present disclosure.
FIG. 1 is a cross-sectional structural schematic view of an electronic atomizing assembly according to some embodiments of the present disclosure.
FIG. 2 is a cross-sectional schematic view of an atomizing base of the electronic atomizing assembly according to some embodiments of the present disclosure.
FIG. 3 is a structural schematic view of an atomizing top base of the electronic atomizing assembly according to a first embodiment of the present disclosure.
FIG. 4 is a structural schematic view of an atomizing bottom base of the electronic atomizing assembly according to the first embodiment the present disclosure.
FIG. 5 is a bottom structural schematic view of the atomizing top base of the electronic atomizing assembly according to a second embodiment of the present disclosure.
FIG. 6 is a structural schematic view of a microgroove structure on a bottom wall of a second groove in the electronic atomizing assembly according to another implementation of the second embodiment of the present disclosure.
FIG. 7 is a top structural schematic view of the atomizing bottom base of the electronic atomizing assembly according to the second embodiment of the present disclosure.
FIG. 8 is a structural schematic view of a microgroove structure on a bottom wall of a third groove and a bottom wall of a fourth groove of the atomizing bottom base of the electronic atomizing assembly according to another implementation of the second embodiment of the present disclosure.
FIG. 9 is a partial structural schematic view of a first side wall of the second groove of the atomizing top base of the electronic atomizing assembly according to a third embodiment of the present disclosure.
FIG. 10 is a partial structural schematic view of a second side wall of the third groove of the atomizing bottom base of the electronic atomizing assembly according to the third embodiment of the present disclosure.
FIG. 11 is a structural schematic view of an electronic atomizing device according to some embodiments of the present disclosure.

### DETAILED DESCRIPTION

The present disclosure is further described in detail below with reference to the accompanying drawings and embodiments. It is specifically noted that the following embodiments are only intended to illustrate the present disclosure and are not intended to limit the scope thereof. Similarly, the following embodiments are merely some rather than all of the embodiments of the present disclosure, and all other embodiments obtained by a person of ordinary skill in the art without the creative efforts shall fall within the protection scope of the present disclosure.

The terms "first", "second", and "third" in the present disclosure are used for descriptive purposes only and should not be construed as indicating or implying relative importance or implicitly indicating the number of technical features indicated. Therefore, features defined by "first", "second", or "third" may explicitly indicate or implicitly include at least one of the features. In the descriptions of the present disclosure, unless otherwise specified, "multiple" means two or more than two, for example, two or three. All directional indications (for example, up, down, left, right, front, back) in the embodiments of the present disclosure are only used for explaining relative position relationships, movement situations, or the like between various components in a specific posture (as shown in the accompanying drawings). If the specific posture changes, the directional indications change accordingly. In the embodiments of the present disclosure, terms "include", "comprise", and any variations thereof are intended to cover non-exclusive inclusion. For example, a process, method, system, product, or device that includes a series of steps or units is not limited to the listed steps or units; and instead, further optionally includes a step or unit that is not listed, or further optionally includes another step or component that is intrinsic to the process, method, product, or device.

Embodiment mentioned in the specification means that particular features, structures, or characteristics described with reference to the embodiment may be included in at least one embodiment of the present disclosure. The term appearing at different positions of the specification may not refer to the same embodiment or an independent or alternative embodiment that is mutually exclusive with another embodiment. A person skilled in the art explicitly or implicitly understands that the embodiments described in the specification may be combined with other embodiments.

An electronic atomizing assembly is provided and includes an atomizing core configured to atomize liquid, an atomizing base, and a liquid storage tank. The atomizing core includes a heating body and an atomizing core body having a porous structure. The atomizing base includes an atomizing top base, an atomizing bottom base, and a microgroove structure. The atomizing top base is cooperated with the atomizing bottom base to form an atomizing cavity, and the atomizing core is fixed between the atomizing top base and the atomizing bottom base. The microgroove structure includes a plurality of microgrooves, wherein the surface of at least one of the atomizing top base and the atomizing bottom base which is configured to be contacted with the atomizing core body is provided with the plurality of microgrooves. The liquid storage tank is sleeved on the atomizing base and configured to store the liquid.

In some embodiments, liquid flowing channel is defined in the atomizing top base, fluidly communicated to the liquid storage tank, and the liquid in the liquid storage tank arrives at the atomizing core through the liquid flowing channel.

In some embodiments, the liquid flowing channel is fluidly communicated to the atomizing cavity, and the liquid is allowed to be guided into the plurality of microgrooves through the liquid following channel.

In some embodiments, the atomizing core includes a first fixing portion, located at one end of the atomizing core; and a second fixing portion, located at the other end of the atomizing core; wherein the first fixing portion and the second fixing portion are fixed between the atomizing top base and the atomizing bottom base, the atomizing core transverses the atomizing cavity, and the microgroove structure is in contact with at least one of the first fixing portion and the second fixing portion.

In some embodiments, the atomizing cavity includes an upper portion, located in the atomizing top base; and a lower portion, located in the atomizing bottom base; wherein the plurality of microgrooves includes a first microgroove; and a third microgroove; wherein the first microgroove and the third microgroove are defined at the surface of the atomizing top base, and located at both sides of the atomizing cavity, respectively.

In some embodiments, the plurality of microgrooves further includes a fifth microgroove; and a sixth microgroove; wherein the fifth microgroove and the sixth microgroove are defined in the atomizing bottom base, and locate at the both sides of the atomizing cavity, respectively.

In some embodiments, one end of each of the plurality of microgrooves is fluidly communicated to the liquid flowing channel, and the other end of each of the plurality of microgrooves is fluidly communicated to the atomizing cavity.

In some embodiments, the number of liquid flowing channels is two, and the two liquid flowing channels are located at both sides of the atomizing cavity, respectively; and both ends of the atomizing core extend into the two liquid flowing channels, respectively.

In some embodiments, the plurality of microgrooves is parallel to each other; or at least two microgrooves defined at the same surface and located at the same side of the atomizing cavity are not parallel to each other.

In some embodiments, the width of each of the plurality of microgrooves is less than 1 mm; and the cross section of each of the plurality of microgrooves has one of a semicircular shape, a rectangular shape, or a triangular shape.

In some embodiments, the atomizing core body is a cotton core or a porous ceramic.

An electronic atomizing device is provided and includes an electronic atomizing assembly in any of the above embodiments and a power supply component.

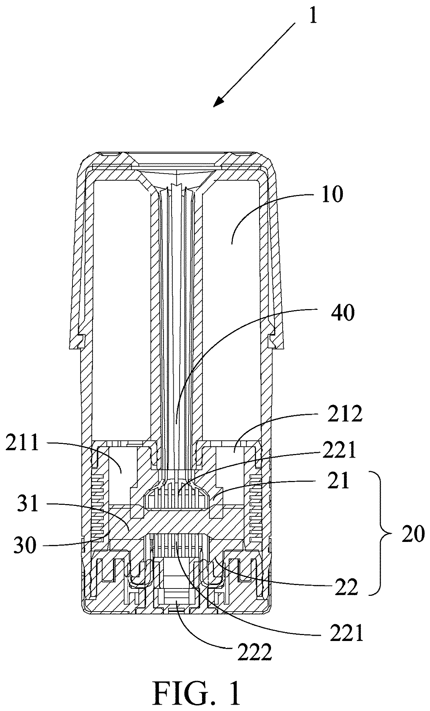

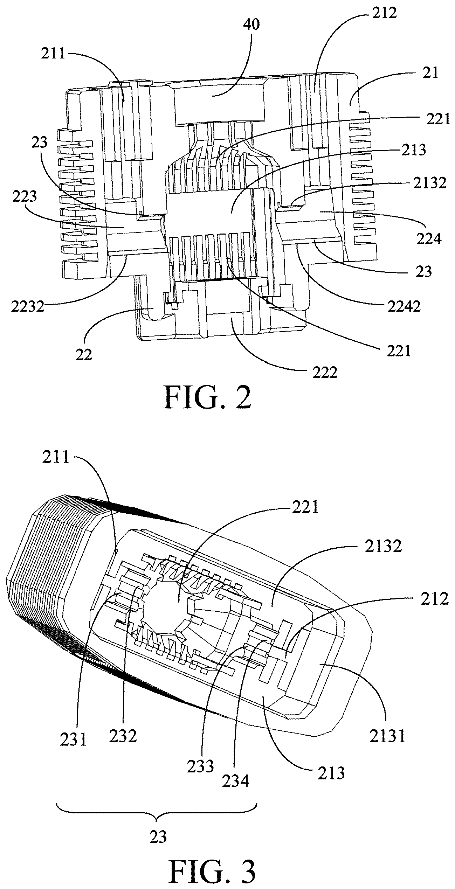

Referring to FIG. 1 and FIG. 2, FIG. 1 is a structural schematic view of an electronic atomizing assembly 1 according to some embodiments of the present disclosure, and FIG. 2 is a cross-sectional schematic view of an atomizing base 20 of the electronic atomizing assembly 1 according to some embodiments of the present disclosure.

The electronic atomizing assembly 1 includes a liquid storage tank 10, an atomizing base 20, and an atomizing core 30. The electronic atomizing assembly 1 may be configured to atomize liquid and generate vapor. The electronic atomizing assembly 1 may be applied to different fields, such as medical treatment, electronic cigarettes, and the like. In an embodiment, the electronic atomizing assembly 1 is applied to an electronic cigarette atomizing device and configured to atomize e-liquid and generate the vapor for an inhaler to inhale, which is taken as an example in all the following embodiments. Certainly, in other embodiments, the electronic atomizing assembly 1 may also be applied to a hair spray device to atomize hair spray for hair styling, or be applied to a medical device for treating upper and lower respiratory system diseases and configured to atomize medical drugs.

The liquid storage tank 10 is sleeved on the atomizing base 20, and is configured to store the e-liquid. The liquid storage tank 10 may be made of aluminum, stainless steel, or other metals allowing the liquid storage tank to store the e-liquid without reacting with the e-liquid to deteriorate the e-liquid. A shape and a size of the liquid storage tank 10 are not limited herein, which may be designed as required.

The atomizing base 20 includes an atomizing top base 21 and an atomizing bottom base 22. The atomizing base 20 may be made of ceramic, stainless steel or other alloys, which is not limited herein but a support function is required. A shape and a size of the atomizing base 20 are not limited herein, which may be designed as required.

The atomizing core 30 is arranged between the atomizing top base 21 and the atomizing bottom base 22. The atomizing core 30 includes an atomizing core body 31 and a heating body, and the atomizing core body 31 is configured to wrap the heating body. The atomizing core body 31 has a porous structure. A material of the atomizing core body 31 is a cotton core or a porous ceramic. Since the atomizing core body 31 has a porous structure, the atomizing core body 31 may form or have a capillary action. After the atomizing core body 31 is in contact with the e-liquid in the liquid storage tank 10, the e-liquid may reach the heating body inside the atomizing core body 31 by passing through the atomizing core body 31, and is atomized by the heating body.

A first groove is provided on the atomizing bottom base 22 to form a lower portion of an atomizing cavity 221. A first through hole is provided on the bottom wall of the atomizing cavity 221 to form an air inlet channel 222. The air inlet channel 222 is fluidly communicated to the atomizing cavity 221, such that the atomizing cavity 221 is fluidly communicated to the outside via the air inlet channel 222. A second through hole and a third through hole are provided on the atomizing top base 21 to form a first liquid flowing channel 211 and a second liquid flowing channel 212, respectively. The e-liquid in the liquid storage tank 10 may be guided to the atomizing core 30 through the first liquid flowing channel 211 and the second liquid flowing channel 212, and be atomized by the atomizing core 30. A fourth through hole is further provided on the atomizing top base 21 to form an upper portion of the atomizing cavity 221. The first liquid flowing channel 211 and the second liquid flowing channel 212 are located on both sides of the atomizing cavity 221.

The electronic atomizing assembly 1 further includes an inhalation channel 40, and the inhalation channel 40 runs through the liquid storage tank 10. The outside is fluidly communicated to the atomizing cavity 221 via the inhalation channel 40. External air enters the atomizing cavity 221 through the air inlet channel 222 to carry the vapor in the atomizing cavity 221, and the vapor is then inhaled by a user through the inhalation channel 40.

Referring to FIG. 2, a second groove 213 is provided on the atomizing top base 21 and configured to accommodate a part of the atomizing bottom base 22, and the atomizing core 30 is arranged in the second groove 213. The first liquid flowing channel 211 and the second liquid flowing channel 212 are provided on the bottom wall 2132 of the second groove 213, to allow the e-liquid to be guided to the atomizing core 30. The upper side of the atomizing cavity 221 is provided on the bottom wall 2132 of the second groove 213, to allow atomized e-liquid to be inhaled by the user.

A third groove 223 and a fourth groove 224 are provided on the atomizing bottom base 22, and are located at both sides of the lower portion of the atomizing cavity 221. The third groove 223 and the fourth groove 224 are through grooves. The third groove 223 and the fourth groove 224 have the same shape and size. The side wall 2231 of the third groove 223 is flush with the side wall 2241 of the fourth groove 224, and the bottom wall 2232 of the third groove 223 is flush with the bottom wall 2242 of the fourth groove 224. The third groove 223 and the fourth groove 224 are fluidly communicated to the atomizing cavity 221, and the depth of the atomizing cavity 221 is greater than the depth of the third groove 223 and the depth of the fourth groove 224. A portion of the atomizing bottom base 22 on which the third groove 223 and the fourth groove 224 are provided is completely accommodated in the second groove 213, and both ends of the atomizing core 30 are received in and engaged with the third groove 223 and the fourth groove 224, respectively.

A microgroove structure 23 is provided on the surface of the atomizing base 20 which is configured to be contacted with the atomizing core body 31. In some embodiments, the microgroove structure 23 may be provided on the atomizing top base 21 and/or the atomizing bottom base 22. The microgroove structure 23 has a guiding action on the e-liquid, and may also finely adjust an amount of the e-liquid entering the atomizing core 30. The microgroove structure 23 includes at least one microgroove.

The atomizing core 30 is abutted against the atomizing top base 21 and the atomizing bottom base 22 to be fixed between the atomizing top base 21 and the atomizing bottom base 22. In an embodiment, the atomizing core 30 may be deformed. For example, the atomizing core 30 is the cotton core. During assembly, the atomizing core 30 is squeezed, a portion on which the microgroove structure 23 is provided has a smaller pressure, and the atomizing core 30 partially extends into the at least one microgroove.

As shown in FIG. 3, FIG. 3 is a structural schematic view of an atomizing top base 21 of the electronic atomizing assembly 1 according to a first embodiment of the present disclosure.

In the first embodiment, the second groove 213 of the atomizing top base 21 includes a first side wall 2131 and a bottom wall 2132. The microgroove structure 23 is provided on the bottom wall 2132 of the second groove 213 of the atomizing top base 21, a part of the microgroove structure 23 is provided between the first liquid flowing channel 211 and the atomizing cavity 221, and an another part of microgroove structure 23 is provided between the second liquid flowing channel 212 and the atomizing cavity 221. The microgroove structure 23 may include one microgroove or multiple microgrooves. For example, the microgroove structure 23 includes four microgrooves. The microgroove structure 23 provided on the bottom wall 2132 of the second groove 213 includes a first microgroove 231, a second microgroove 232, a third microgroove 233, and a fourth microgroove 234. The first microgroove 231, the second microgroove 232, the third microgroove 233, and the fourth microgroove 234 have the same depths and widths.

The first microgroove 231 and the second microgroove 232 are provided on the bottom surface between the first liquid flowing channel 211 and the atomizing cavity 221, and the first microgroove 231 and the second microgroove 232 are in parallel. One end of each of the first microgroove 231 and the second microgroove 232 is fluidly communicated to the first liquid flowing channel 211, and the other end of each of the first microgroove 231 and the second microgroove 232 extends towards the atomizing cavity 221. In an embodiment, the other end of each of the first microgroove 231 and the second microgroove 232 is fluidly communicated to the atomizing cavity 221. When the other end of each of the first microgroove 231 and the second microgroove 232 is fluidly communicated to the atomizing cavity 221, the e-liquid may be better guided to the heating body, but the liquid leakage problem of the atomizing cavity 221 may occur. When the other end of each of the first microgroove 231 and the second microgroove 232 extends towards the atomizing cavity 221 and is not fluidly communicated to the atomizing cavity 221, a liquid-guiding effect is worse than that of a case where the other end of each of the first microgroove 231 and the second microgroove 232 is fluidly communicated to the atomizing cavity 221, but the liquid leakage problem of the atomizing cavity 221 may be avoided.

The third microgroove 233 and the fourth microgroove 234 are provided between the second liquid flowing channel 212 and the atomizing cavity 221, and the third microgroove 233 and the fourth microgroove 234 are in parallel. One end of each of the third microgroove 233 and the fourth microgroove 234 is fluidly communicated to the second liquid flowing channel 212, and the other end of each of the third microgroove 233 and the fourth microgroove 234 extends towards the atomizing cavity 221. In an embodiment, the other end of each of the third microgroove 233 and the fourth microgroove 234 is fluidly communicated to the atomizing cavity 221. When the other end of each of the third microgroove 233 and the fourth microgroove 234 is fluidly communicated to the atomizing cavity 221, the e-liquid may be better guided to the heating body, but the liquid leakage problem of the atomizing cavity 221 may occur. When the other end of each of the third microgroove 233 and the fourth microgroove 234 extends towards the atomizing cavity 221 and is not fluidly communicated to the atomizing cavity 221, the liquid guiding effect is worse than that of a case where the other end of each of the third microgroove 233 and the fourth microgroove 234 is fluidly communicated to the atomizing cavity 221, but the liquid leakage problem of the atomizing cavity 221 may be avoided.

The first microgroove 231 and the third microgroove 233 are collinear, and the second microgroove 232 and the fourth microgroove 234 are collinear.

The microgroove structure 23 on the bottom wall 2132 of the second groove 213 of the atomizing top base 21 may only include one microgroove 231, and the microgroove 231 may be provided between the first liquid flowing channel 211 and the atomizing cavity 221, or may be provided between the second liquid flowing channel 212 and the atomizing cavity 221. The microgroove structure 23 on the bottom wall 2132 of the second groove 213 of the atomizing top base 21 may include multiple microgrooves, and the number of microgrooves provided on the bottom surface between the first liquid flowing channel 211 and the atomizing cavity 221 may be the same with of different from the number of microgrooves provided on the bottom surface between the second liquid flowing channel 212 and the atomizing cavity 221. The microgrooves provided between the first liquid flowing channel 211 and the atomizing cavity 221 and the microgrooves provided between the second liquid flowing channel 212 and the atomizing cavity 221 may or may not be collinear. The bottoms of the microgrooves provided between the liquid flowing channel 211 and the atomizing cavity 221 may or may not be flush with the bottoms of the microgrooves provided between the second liquid flowing channel 212 and the atomizing cavity 221. The multiple microgrooves in the microgroove structure 23 may be linear or non-linear.

The microgrooves 231 to 234 may have the same shape or different shapes. The shapes of the microgrooves 231 to 234 may be one or a combination of a straight line, a curve, a T-shape, and an I-shape. In some embodiments, the liquid flowing channel 211 or the liquid flowing channel 212 is fluidly communicated to the atomizing cavity 221 via the microgrooves 231 to 234. In this way, a capillary liquid-guiding effect may be enhanced.

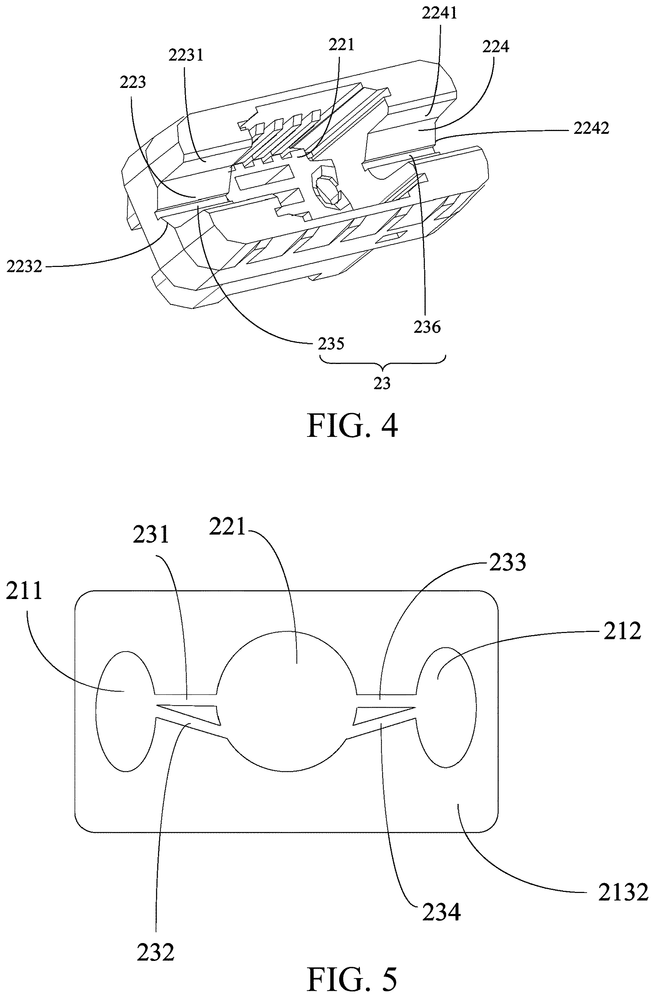

As shown in FIG. 4, FIG. 4 is a structural schematic view of the atomizing bottom base 22 of the electronic atomizing assembly according to the first embodiment the present disclosure.

In the first embodiment, the third groove 223 on the atomizing bottom base 22 includes a second side wall 2231 and a bottom wall 2232, and the fourth groove 224 on the atomizing bottom base 22 includes a third side wall 2241 and a bottom wall 2242. A microgroove structure 23 is provided on the bottom wall 2232 of the third groove 223 and the bottom wall 2242 of the fourth groove 224 of the atomizing bottom base 22. The microgroove structure 23 may include one microgroove or multiple microgrooves. A fifth microgroove 235 is provided on the bottom wall 2232 of the third groove 223, one end of the fifth microgroove 235 is fluidly communicated to the atomizing cavity 221, and the other end of the fifth microgroove 235 extends away from the atomizing cavity 221. In an embodiment, the fifth microgroove 235 is a through groove. A sixth microgroove 236 is provided on the bottom wall 2242 of the fourth groove 224. One end of the sixth microgroove 236 is fluidly communicated to the atomizing cavity 221, and the other end of the sixth microgroove 236 extends away from the atomizing cavity 221. In an embodiment, the sixth microgroove 236 is a through groove. The fifth microgroove 235 and the sixth microgroove 236 have the same depth and the same width, and the fifth microgroove 235 and the sixth microgroove 236 are collinear. When the fifth microgroove 235 and the sixth microgroove 236 are through grooves, since the portion of the atomizing bottom base 22 on which the third groove 223 and the fourth groove 224 are provided is completely accommodated in the second groove 213 of the atomizing top base 21, the fifth microgroove 235 and the sixth microgroove 236 are fluidly communicated to the first side wall 2131 of the second groove 213.

Multiple microgrooves may be provided on the bottom wall 2232 of the third groove 223 of the atomizing bottom base 22, and the multiple microgrooves are parallel to each other. Multiple microgrooves may be provided on the bottom wall 2242 of the fourth groove 224 of the atomizing bottom base 22, and the multiple microgrooves are parallel to each other. The number of microgrooves on the bottom wall 2232 of the third groove 223 may be the same with or different from the number of microgrooves on the bottom wall 2242 of the fourth groove 224. The microgrooves on the bottom wall 2232 of the third groove 223 and the microgrooves on the bottom wall 2242 of the fourth groove 224 may or may not be collinear. The bottoms of the microgrooves on the bottom wall 2232 of the third groove 223 may or may not be flush with the bottoms of the microgrooves on the bottom wall 2242 of the fourth groove 224. The microgrooves provided on the bottom wall 2232 of the third groove 223 and the bottom wall 2242 of the fourth groove 224 of the atomizing bottom base 22 may be linear or non-linear.

The microgrooves 235 and 236 may have the same shape or different shapes. The shapes of the microgrooves 235 and 236 may be one or a combination of the straight line, the curve, the T-shape, and the I-shape. In an embodiment, the microgrooves 235 and 236 are through grooves. In this way, the capillary liquid-guiding effect may be enhanced.

In other implementations, the microgroove structure 23 may only be provided on the bottom wall 2132 of the second groove 213 of the atomizing top base 21, or the microgroove structure 23 may only be provided on the bottom wall 2232 of the third groove 223 and the bottom wall 2242 of the fourth groove 224 of the atomizing bottom base 22, or the microgroove structure 23 may be provided on both the bottom wall 2132 of the second groove 213 of the atomizing base 21, and the bottom wall 2232 of the third groove 223 and the bottom wall 2242 of the fourth groove 224 of the atomizing bottom base 22.

As shown in FIG. 5, FIG. 5 is a bottom structural schematic view of the atomizing top base 21 of the electronic atomizing assembly 1 according to a second embodiment of the present disclosure.

In the second embodiment, a first microgroove 231 and a second microgroove 232 are provided on the bottom wall 2132 of the second groove 213 of the atomizing top base 21 and located between the first liquid flowing channel 211 and the atomizing cavity 221. One end of the first microgroove 231 is fluidly communicated to the first liquid flowing channel 211, and the other end of the first microgroove 231 extends towards the atomizing cavity 221, and the other end of the first microgroove 231 may or may not be fluidly communicated to the atomizing cavity 221. One end of the second microgroove 232 is fluidly communicated to the first liquid flowing channel 211, and the other end of the second microgroove 232 extends towards the atomizing cavity 221. The other end of the second microgroove 232 may or may not be fluidly communicated to the atomizing cavity 221. The first microgroove 231 is intersected with the second microgroove 232, and an intersection point there between may be fluidly communicated to the atomizing cavity 221, or may be fluidly communicated to the first liquid flowing channel 211, or may be provided between the atomizing cavity 221 and the first liquid flowing channel 211. The first microgroove 231 and the second microgroove 232 have the same depth and the same width.

A third microgroove 233 and a fourth microgroove 234 are provided on the bottom wall 2132 of the second groove 213 of the atomizing top base 21 and located between the second liquid flowing channel 212 and the atomizing cavity 221. One end of the third microgroove 233 is fluidly communicated to the second liquid flowing channel 212, and the other end extends towards the atomizing cavity 221, and the other end of the third microgroove 233 may or may not be fluidly communicated to the atomizing cavity 221. One end of the fourth microgroove 234 is fluidly communicated to the second liquid flowing channel 212, and the other end extends towards the atomizing cavity 221. The other end of the fourth microgroove 234 may or may not be fluidly communicated to the atomizing cavity 221. The third microgroove 233 is intersected with the fourth microgroove 234, and an intersection point there between may be fluidly communicated to the atomizing cavity 221, or may be fluidly communicated to the second liquid flowing channel 212, or may be provided between the atomizing cavity 221 and the second liquid flowing channel 212. The third microgroove 233 and the fourth microgroove 234 have the same depth and the same width.

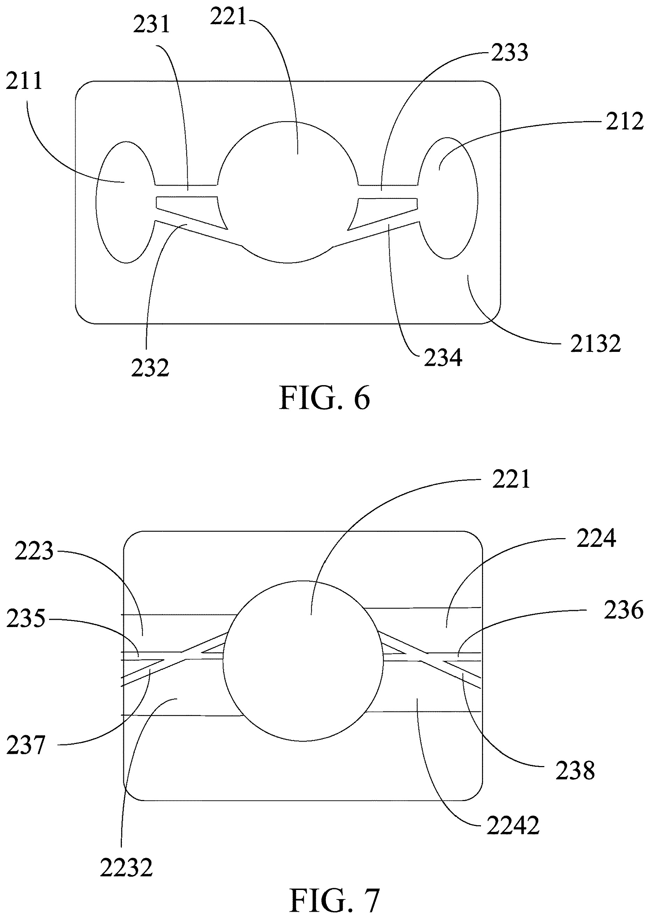

As shown in FIG. 6, FIG. 6 is a structural schematic view of the microgroove structure 23 on the bottom wall 2132 of the second groove 213 in the electronic atomizing assembly 1 according to another implementation of the second embodiment the present disclosure.

In another implementation, the extending direction of the first microgroove 231 is intersected with the extending direction of the second microgroove 232. The first microgroove 231 is spaced from the second microgroove 232. The extending direction of the third microgroove 233 is intersected with the extending direction of the fourth microgroove 234. The third microgroove 233 is spaced from the fourth microgroove 234.

As shown in FIG. 7, FIG. 7 is a top structural schematic view of the atomizing bottom base 22 of the electronic atomizing assembly 1 according to the second embodiment of the present disclosure.

In the second embodiment, the fifth microgroove 235 and a seventh microgroove 237 are provided on the bottom wall 2232 of the third groove 223 of the atomizing bottom base 22. One end of the fifth microgroove 235 is fluidly communicated to an atomizing cavity 221, and the other end of the fifth microgroove 235 extends away from the atomizing cavity 221. One end of the seventh microgroove 237 is fluidly communicated to the atomizing cavity 221, and the other end of the seventh microgroove 237 extends away from the atomizing cavity 221. The fifth microgroove 235 is intersected with the seventh microgroove 237, and an intersection point there between may be fluidly communicated to the atomizing cavity 221, or may be fluidly communicated to the first side wall 2131 of the second groove 213, or may be provided between the atomizing cavity 221 and the first side wall 2131 of the second groove 213 (the portion of the atomizing bottom base 22 on which the third groove 223 and the fourth groove 224 are provided is completely accommodated in the second groove 213). The fifth microgroove 235 and the seventh microgroove 237 have the same depth and the same width.

The sixth microgroove 236 and an eighth microgroove 238 are provided on the bottom wall 2242 of the fourth groove 224 of the atomizing bottom base 22. One end of the sixth microgroove 236 is fluidly communicated to the atomizing cavity 221, and the other end of the sixth microgroove 236 extends away from the atomizing cavity 221. One end of the eighth microgroove 238 is fluidly communicated to the atomizing cavity 221, and the other end of the eighth microgroove 238 extends away from the atomizing cavity 221. The sixth microgroove 236 is intersected with the eighth microgroove 238. An intersection point there between may be fluidly communicated to the atomizing cavity 221, or may be fluidly communicated to the first side wall 2131 of the second groove 213, or may be provided between the atomizing cavity 221 and the first side wall 2131 of the second groove 213 (the portion of the atomizing bottom base 22 on which the third groove 223 and the fourth groove 224 are provided is completely accommodated in the second groove 213). The sixth microgroove 236 and the eighth microgroove 238 have the same depth and the same width.

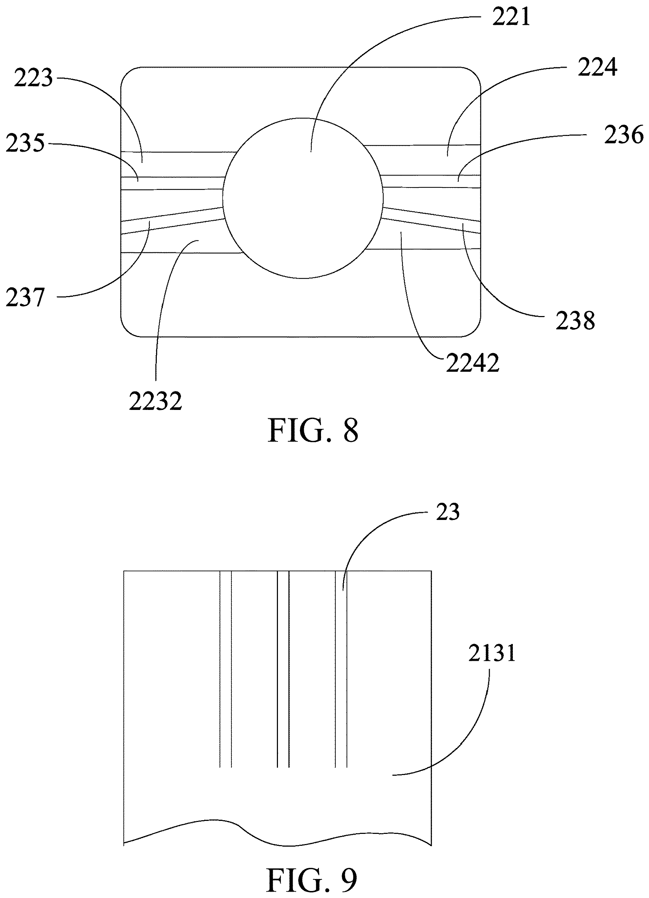

As shown in FIG. 8, FIG. 8 is a structural schematic view of the microgroove structure 23 on the bottom wall 2232 of the third groove 223 and the bottom wall 2242 of the fourth groove 224 of the atomizing bottom base 22 of the electronic atomizing assembly 1 according to another implementation of the second embodiment of the present disclosure.

In another implementation, the extending direction of the fifth microgroove 235 is intersected with the extending direction of the seventh microgroove 237. The fifth microgroove 235 is spaced from the seventh microgroove 237. The extending direction of the sixth microgroove 236 is intersected with the extending direction of the eighth microgroove 238. The sixth microgroove 236 is spaced from the eighth microgroove 238.

In other implementations, more than two microgrooves are provided on the bottom wall 2132 of the second groove 213 of the atomizing top base 21 and are located between the first liquid flowing channel 211 and the atomizing cavity 221. The more than two microgrooves may be intersected with each other, or the more than two microgrooves are spaced from each other and the extending directions of which may be intersected with each other. More than two microgrooves are provided between the second liquid flowing channel 212 and the atomizing cavity 221. The more than two microgrooves may be intersected with each other, or the more than two microgrooves may be spaced from each other and the extending directions of which may be intersected with each other. More than two microgrooves are provided on the bottom wall 2232 of the third groove 223 of the atomizing bottom base 22. The more than two microgrooves may be intersected with each other, or the more than two microgrooves may be spaced from each other and the extending directions of which may be intersected with each other. More than two microgrooves are provided on the bottom wall 2242 of the fourth groove 224 of the atomizing bottom base 22. The more than two microgrooves may be intersected with each other, or the more than two microgrooves may be spaced from each other and the extending directions of which may be intersected with each other.

As shown in FIG. 9, FIG. 9 is a partial structural schematic view of the first side wall 2131 of the second groove 213 of the atomizing top base 21 of the electronic atomizing assembly 1 according to a third embodiment of the present disclosure.

In the third embodiment, the microgroove structure 23 is provided on the bottom wall 2132 of the second groove 213 of the atomizing top base 21, located between the first liquid flowing channel 211 and the atomizing cavity 221 and between the second liquid flowing channel 212 and the atomizing cavity 221. The microgroove structure 23 is also provided on the first side wall 2131 of the second groove 213 of the atomizing top base 21.

The microgroove structure 23 is provided on the bottom wall 2132 of the second groove 213 of the atomizing base 21, and the microgroove structure 23 is configured in the same way as those in the first embodiment and the second embodiment, which is not repeated herein.

The microgroove structure 23 provided on the first side wall 2131 of the second groove 213 of the atomizing top base 21 includes multiple microgrooves. The multiple microgrooves may extend in the direction of the axis of the electronic atomizing assembly 1, or may extend in a direction perpendicular to the direction of the axis of the electronic atomizing assembly 1. The multiple microgrooves may be parallel to each other, or the multiple microgrooves may be intersected with each other. The multiple microgrooves may be linear or non-linear.

The microgrooves provided on the first side wall 2131 of the second groove 213 may be fluidly communicated to the microgrooves provided on the bottom wall 2132 between the first liquid flowing channel 211 and the atomizing cavity 221. The microgrooves provided on the first side wall 2131 of the second groove 213 may be fluidly communicated to the microgrooves provided on the bottom wall 2132 between the second liquid flowing channel 212 and the atomizing cavity 221.

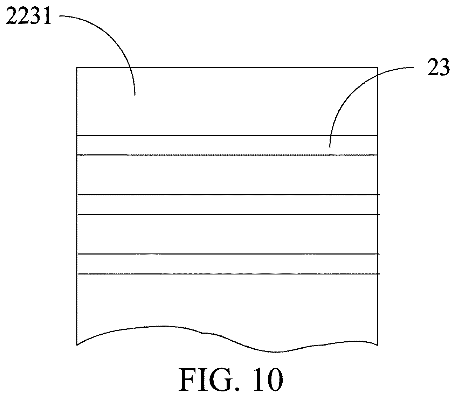

As shown in FIG. 10, FIG. 10 is a partial structural schematic view of the second side wall 2231 of the third groove 223 of the atomizing bottom base 22 of the electronic atomizing assembly 1 according to the third embodiment of the present disclosure.

In the third embodiment, the microgroove structure 23 is provided on the bottom wall 2232 of the third groove 223 and the bottom wall 2242 of the fourth groove 224 of the atomizing bottom base 22, and the microgroove structure 23 is configured in the same way as those in the first embodiment and the second embodiment, which is not repeated herein. In addition, the microgroove structure 23 is also provided on the second side wall 2231 of the third groove 223 and the third side wall 2241 of the fourth groove 224.

The microgroove structure 23 provided on the second side wall 2231 of the third groove 223 and the third side wall 2241 of the fourth groove 224 includes multiple microgrooves. The multiple microgrooves may be parallel to each other, or the multiple microgrooves may be intersected with each other. The multiple microgrooves may extend towards the bottom wall 2232 or the bottom wall 2242 from an opening of the third groove 223 or an opening of the fourth groove 224, or extend away from the atomizing cavity 221 from the atomizing cavity 221. The multiple microgrooves may be linear or non-linear.

The microgrooves provided on the second side wall 2231 of the third groove 223 may be fluidly communicated to the microgrooves on the first side wall 2131 of the second groove 213. The microgrooves provided on the third side wall 2241 of the fourth groove 224 may be fluidly communicated to the microgrooves provided on the first side wall 2131 of the second groove 213.

In the invention, the width of the microgroove structure 23 (i.e., each of the plurality of microgrooves) is less than 1 mm. In an embodiment, the width ranges from 0.2 mm to 0.5 mm. The cross section of the microgroove structure 23 (i.e., each of the plurality of microgrooves) has a semicircular shape, a rectangular shape, a triangular shape, or other shapes, which is not limited herein as long as a fine adjustment for the liquid-supplying amount is achieved. In the first embodiment, the second embodiment, and the third embodiment, the microgroove structure 23 on the atomizing top base 21 and the microgroove structure 23 on the atomizing bottom base 22 are provided in different manners. A manner for configuring the microgroove structure 23 on the atomizing base 21 may be randomly combined with a manner for configuring the microgroove structure 23 on the atomizing bottom base 22, which is not limited herein.

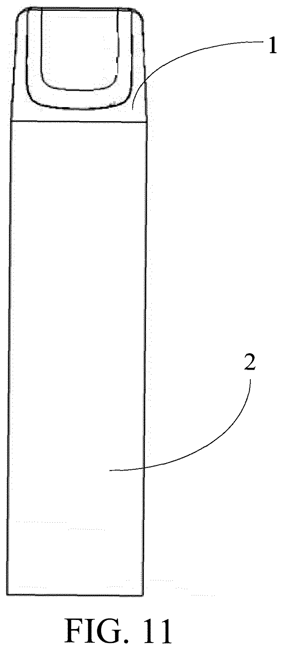

As shown in FIG. 11, FIG. 11 is a structural schematic view of an electronic atomizing device according to some embodiments of the present disclosure.

The electronic atomizing device includes an electronic atomizing assembly 1 and a power supply component 2. The power supply component 2 is configured to supply power to the electronic atomizing assembly 1 to allow the electronic atomizing assembly 1 operate. The electronic atomizing assembly 1 is the electronic atomizing assembly 1 in any of the foregoing embodiments.

In the present disclosure, since the microgroove structure 23 is provided on the surface of the atomizing base 20 which is configured to be contacted with the atomizing core body 31, the liquid-supplying amount of the e-liquid entering the atomizing core 30 may be allowed to be finely adjusted. In this way, the precise control for the liquid-supplying amounts of the different products may be achieved, and the liquid leakage problem of the atomizing core 30 caused by the liquid-supplying amount being excessively large, or the problems such as the e-liquid explosion of the atomizing core body 30 or the burnt flavor caused by the liquid-supplying amount being an excessively small may be avoided. Therefore, the performance of the electronic atomizing device may be enhanced.

The above descriptions are merely a part of the embodiments of the present disclosure, and the protection scope of the present disclosure is not limited thereto. The scope of protection of the present invention is defined by the appended claims.

## Claims

1. An electronic atomizing assembly (1), comprising:
an atomizing core (30), configured to atomize liquid and comprising:
a heating body; and
an atomizing core body (31), having a porous structure;
an atomizing base (20), comprising:
an atomizing top base (21);
an atomizing bottom base (22); wherein the atomizing top base (21) is cooperated with the atomizing bottom base (22) to form an atomizing cavity (221), the atomizing core (30) is fixed between the atomizing top base (21) and the atomizing bottom base (22); and
a microgroove structure (23), comprising a plurality of microgrooves, wherein the surface of at least one of the atomizing top base (21) and the atomizing bottom base (22) which is configured to be contacted with the atomizing core body (31) is provided with the plurality of microgrooves; and
a liquid storage tank (10), sleeved on the atomizing base (20), and configured to store the liquid;
**characterized in that** the width of each of the plurality of microgrooves is less than 1 mm.

2. The electronic atomizing assembly (1) according to claim 1, wherein a liquid flowing channel (211, 212) is defined in the atomizing top base (21), fluidly communicated to the liquid storage tank (10), and the liquid in the liquid storage tank (10) arrives at the atomizing core (30) through the liquid flowing channel (211, 212).

3. The electronic atomizing assembly (1) according to claim 2, wherein the liquid flowing channel (211, 212) is fluidly communicated to the atomizing cavity (221), and the liquid is allowed to be guided into the plurality of microgrooves through the liquid following channel (211, 212).

4. The electronic atomizing assembly (1) according to claim 1, wherein the atomizing core (30) comprises:
a first fixing portion, located at one end of the atomizing core (30); and
a second fixing portion, located at the other end of the atomizing core (30);
wherein the first fixing portion and the second fixing portion are fixed between the atomizing top base (21) and the atomizing bottom base (22), the atomizing core (30) transverses the atomizing cavity (221), and the microgroove structure (23) is in contact with at least one of the first fixing portion and the second fixing portion.

5. The electronic atomizing assembly (1) according to claim 4, wherein the atomizing cavity (221) comprises:
an upper portion, located in the atomizing top base (21); and
a lower portion, located in the atomizing bottom base (22);
wherein the plurality of microgrooves comprises:
a first microgroove (231); and
a third microgroove (233);
wherein the first microgroove (231) and the third microgroove (233) are defined at the surface of the atomizing top base (21), and located at both sides of the atomizing cavity (221), respectively.

6. The electronic atomizing assembly (1) according to claim 5, wherein the plurality of microgrooves further comprises:
a fifth microgroove (235); and
a sixth microgroove (236);
wherein the fifth microgroove (235) and the sixth microgroove (236) are defined in the atomizing bottom base (22), and located at the both sides of the atomizing cavity (221), respectively.

7. The electronic atomizing assembly (1) according to any one of claims 2-6, wherein one end of each of the plurality of microgrooves is fluidly communicated to the liquid flowing channel (211, 212), and the other end of each of the plurality of microgrooves is fluidly communicated to the atomizing cavity (221).

8. The electronic atomizing assembly (1) according to claim 2, wherein the number of liquid flowing channels (211, 212) is two, and the two liquid flowing channels (211, 212) are located at both sides of the atomizing cavity (221), respectively; and both ends of the atomizing core (30) extend into the two liquid flowing channels (211, 212), respectively.

9. The electronic atomizing assembly (1) according to any one of claims 1-8, wherein the plurality of microgrooves is parallel to each other; or
at least two microgrooves defined at the same surface and located at the same side of the atomizing cavity (221) are not parallel to each other.

10. The electronic atomizing assembly (1) according to claim 1, wherein the cross section of each of the plurality of microgrooves has one of a semicircular shape, a rectangular shape, and a triangular shape.

11. The electronic atomizing assembly (1) according to claim 1, wherein the atomizing core body (31) is a cotton core or a porous ceramic.

12. An electronic atomizing device, **characterized by** comprising:
an electronic atomizing assembly (1) according to any one of claims 1-11; and
a power supply component (2).

## Patentansprüche

1. Elektronische Zerstäubungsanordnung (1), das folgendes umfasst:
einen Zerstäubungskern (30), der zum Zerstäuben von Flüssigkeit konfiguriert ist und der umfasst:
ein Heizmodul; und
ein Zerstäubungskernkörper (31) mit poröser Struktur;
eine Zerstäubungsbasis (20), die umfasst:
eine obere Zerstäubungsbasis (21);
eine untere Zerstäubungsbasis (22);
wobei die obere Zerstäubungsbasis (21) mit der unteren Zerstäubungsbasis (22) zusammenwirkt, um eine Zerstäubungskammer (221) zu bilden, der Zerstäubungskern (30) zwischen der oberen Zerstäubungsbasis (21) und der unteren Zerstäubungsbasis (22) fixiert ist; und
eine Mikrorillenstruktur (23), die eine Vielzahl von Mikrorillen umfasst, wobei die Oberfläche von mindestens einer der oberen Zerstäubungsbasis (21) und der unteren Zerstäubungsbasis (22), die mit dem Zerstäubungskernkörper (31) in Kontakt gebracht werden soll, mit der genannten Vielzahl von Mikrorillen versehen ist; und
einen Flüssigkeitsspeichertank (10), der über die Zerstäubungsbasis (20) geschoben ist und zum Aufbewahren der Flüssigkeit konfiguriert ist;
wobei die Breite jeder einzelnen Mikrorille der Vielzahl von Mikrorillen kleiner als 1 mm ist.

2. Die elektronische Zerstäubungsanordnung (1) gemäß Anspruch 1, wobei ein Flüssigkeitsflusskanal (211, 212) in der oberen Zerstäubungsbasis (21) definiert ist, der mit dem Flüssigkeitsspeichertank (10) fluid in Verbindung steht, und die im Flüssigkeitsspeichertank (10) enthaltene Flüssigkeit über den Flüssigkeitsflusskanal (211, 212) zum Zerstäubungskern (30) geleitet wird.

3. Die elektronische Zerstäubungsanordnung (1) gemäß Anspruch 2, wobei der Flüssigkeitsflusskanal (211, 212) fluid in Verbindung mit der Zerstäubungskammer (221) steht, und die Flüssigkeit über den Flüssigkeitsflusskanal (211, 212) in die Vielzahl von Mikrorillen geleitet wird.

4. Die elektronische Zerstäubungsanordnung (1) gemäß Anspruch 1,
wobei der Zerstäubungskern (30) umfasst:
einen ersten Befestigungsabschnitt, der an einem Ende des Zerstäubungskerns (30) angeordnet ist; und
einen zweiten Befestigungsabschnitt, der am anderen Ende des Zerstäubungskerns (30) angeordnet ist;
wobei der erste Befestigungsabschnitt und der zweite Befestigungsabschnitt zwischen der oberen Zerstäubungsbasis (21) und der unteren Zerstäubungsbasis (22) fixiert sind, der Zerstäubungskern (30) die Zerstäubungskammer (221) durchquert, und die Mikrorillenstruktur (23) mit mindestens einem der ersten und zweiten Befestigungsabschnitte in Kontakt steht.

5. Die elektronische Zerstäubungsanordnung (1) gemäß Anspruch 4,
wobei die Zerstäubungskammer (221) umfasst:
einen oberen Teil, der in der oberen Zerstäubungsbasis (21) angeordnet ist; und
einen unteren Teil, der in der unteren Zerstäubungsbasis (22) angeordnet ist;
wobei die Vielzahl von Mikrorillen umfasst:
eine erste Mikrorille (231); und
eine dritte Mikrorille (233);
wobei die erste Mikrorille (231) und die dritte Mikrorille (233) an der Oberfläche der oberen Zerstäubungsbasis (21) definiert sind und sich jeweils an beiden Seiten der Zerstäubungskammer (221) befinden.

6. Die elektronische Zerstäubungsanordnung (1) gemäß Anspruch 5,
wobei die Vielzahl von Mikrorillen ferner umfasst:
eine fünfte Mikrorille (235); und
eine sechste Mikrorille (236);
wobei die fünfte Mikrorille (235) und die sechste Mikrorille (236) in der unteren Zerstäubungsbasis (22) definiert sind und sich jeweils an beiden Seiten der Zerstäubungskammer (221) befinden.

7. Die elektronische Zerstäubungsanordnung (1) gemäß einem der Ansprüche 2 bis 6, wobei ein Ende jeder Mikrorille unter der Vielzahl von Mikrorillen fluid in Verbindung mit dem Flüssigkeitsflusskanal (211, 212) steht, und das andere Ende jeder Mikrorille unter der Vielzahl von Mikrorillen fluid in Verbindung mit der Zerstäubungskammer (221) steht.

8. Die elektronische Zerstäubungsanordnung (1) gemäß Anspruch 2, wobei die Anzahl der Flüssigkeitsflusskanäle (211, 212) zwei beträgt, die beiden Flüssigkeitsflusskanäle (211, 212) sich jeweils an beiden Seiten der Zerstäubungskammer (221) befinden; und beide Enden des Zerstäubungskerns (30) jeweils in die beiden Flüssigkeitsflusskanäle (211, 212) hineinragen.

9. Die elektronische Zerstäubungsanordnung (1) gemäß einem der Ansprüche 1 bis 8, wobei die Vielzahl von Mikrorillen parallel zueinander verläuft;
oder mindestens zwei Mikrorillen, die auf derselben Oberfläche definiert sind und sich auf derselben Seite der Zerstäubungskammer (221) befinden, nicht parallel zueinander verlaufen.

10. Die elektronische Zerstäubungsanordnung (1) gemäß Anspruch 1, wobei der Querschnitt jeder Mikrorille unter der Vielzahl von Mikrorillen eine der folgenden Formen aufweist: halbkreisförmig, rechteckig oder dreieckig.

11. Die elektronische Zerstäubungsanordnung (1) gemäß Anspruch 1, wobei der Zerstäubungskernkörper (31) aus einer Baumwollkerneinlage oder aus poröser Keramik besteht.

12. Elektronisches Zerstäubungsgerät, **dadurch gekennzeichnet, dass** es umfasst:
eine elektronische Zerstäubungsanordnung (1) gemäß einem der Ansprüche 1 bis 11; und
ein Stromversorgungsmodul (2).

## Revendications

1. Ensemble électronique d'atomisation (1), comprenant :
un noyau d'atomisation (30), configuré pour atomiser un liquide et comprenant :
un corps de chauffage, et
un corps de noyau d'atomisation (31) présentant une structure poreuse ;
une base d'atomisation (20), comprenant :
une base supérieure d'atomisation (21),
une base inférieure d'atomisation (22), la base supérieure d'atomisation (21) coopérant avec la base inférieure d'atomisation (22) afin de former une cavité d'atomisation (221), le noyau d'atomisation (30) étant fixé entre la base supérieure d'atomisation (21) et la base inférieure d'atomisation (22), et
une structure de micro-rainures (23), comprenant une pluralité de micro-rainures, la surface d'au moins l'une de la base supérieure d'atomisation (21) et de la base inférieure d'atomisation (22), qui est configurée pour être en contact avec le corps de noyau d'atomisation (31), étant pourvue de ladite pluralité de micro-rainures ; et
un réservoir de liquide (10), manchonné sur la base d'atomisation (20) et configuré pour stocker le liquide ;
**caractérisé en ce que** la largeur de chaque micro-rainure parmi la pluralité de micro-rainures est inférieure à 1 mm.

2. L'ensemble électronique d'atomisation (1) selon la revendication 1, dans lequel un canal d'écoulement de liquide (211, 212) est défini dans la base supérieure d'atomisation (21), en communication fluide avec le réservoir de liquide (10), et le liquide contenu dans le réservoir de liquide (10) est acheminé vers le noyau d'atomisation (30) à travers le canal d'écoulement de liquide (211, 212).

3. L'ensemble électronique d'atomisation (1) selon la revendication 2, dans lequel le canal d'écoulement de liquide (211, 212) est en communication fluide avec la cavité d'atomisation (221), et le liquide est acheminé dans la pluralité de micro-rainures à travers le canal d'écoulement de liquide (211, 212).

4. L'ensemble électronique d'atomisation (1) selon la revendication 1,
dans lequel le noyau d'atomisation (30) comprend :
une première partie de fixation, située à une extrémité du noyau d'atomisation (30) ; et
une seconde partie de fixation, située à l'autre extrémité du noyau d'atomisation (30) ;
dans laquelle la première partie de fixation et la seconde partie de fixation sont fixées entre la base supérieure d'atomisation (21) et la base inférieure d'atomisation (22), le noyau d'atomisation (30) traversant la cavité d'atomisation (221), et la structure de micro-rainures (23) étant en contact avec au moins l'une de la première partie de fixation et de la seconde partie de fixation.

5. L'ensemble électronique d'atomisation (1) selon la revendication 4,
dans lequel la cavité d'atomisation (221) comprend :
une portion supérieure, située dans la base supérieure d'atomisation (21) ; et
une portion inférieure, située dans la base inférieure d'atomisation (22) ;
dans laquelle la pluralité de micro-rainures comprend : une première micro-rainure (231) ; et une troisième micro-rainure (233) ;
la première micro-rainure (231) et la troisième micro-rainure (233) étant définies à la surface de la base supérieure d'atomisation (21), et situées respectivement de part et d'autre de la cavité d'atomisation (221).

6. L'ensemble électronique d'atomisation (1) selon la revendication 5,
dans laquelle la pluralité de micro-rainures comprend en outre :
une cinquième micro-rainure (235) ; et
une sixième micro-rainure (236) ;
dans laquelle la cinquième micro-rainure (235) et la sixième micro-rainure (236) sont définies dans la base inférieure d'atomisation (22), et situées respectivement de part et d'autre de la cavité d'atomisation (221).

7. L'ensemble électronique d'atomisation (1) selon l'une quelconque des revendications 2 à 6, dans lequel chaque micro-rainure parmi la pluralité de micro-rainures a une extrémité en communication fluide avec le canal d'écoulement de liquide (211, 212), et l'autre extrémité de chaque micro-rainure parmi la pluralité de micro-rainures est en communication fluide avec la cavité d'atomisation (221).

8. L'ensemble électronique d'atomisation (1) selon la revendication 2, dans lequel le nombre de canaux d'écoulement de liquide (211, 212) est de deux, les deux canaux d'écoulement de liquide (211, 212) étant situés respectivement de part et d'autre de la cavité d'atomisation (221) ; et les deux extrémités du noyau d'atomisation (30) s'étendent respectivement dans les deux canaux d'écoulement de liquide (211, 212).

9. L'ensemble électronique d'atomisation (1) selon l'une quelconque des revendications 1 à 8, dans lequel la pluralité de micro-rainures est parallèle entre elles ; ou au moins deux micro-rainures définies sur la même surface et situées du même côté de la cavité d'atomisation (221) ne sont pas parallèles entre elles.

10. L'ensemble électronique d'atomisation (1) selon la revendication 1, dans lequel la section transversale de chaque micro-rainure parmi la pluralité de micro-rainures présente l'une des formes suivantes : semi-circulaire, rectangulaire ou triangulaire.

11. L'ensemble électronique d'atomisation (1) selon la revendication 1, dans lequel le noyau d'atomisation (31) est en coton ou en céramique poreuse.

12. un dispositif électronique d'atomisation, **caractérisé en ce qu'**il comprend :
un ensemble électronique d'atomisation (1) selon l'une quelconque des revendications 1 à 11 ; et
un module d'alimentation électrique (2).
